# EUROPÄISCHE PATENTSCHRIFT

(11) **EP 1 478 776 B1**
(45) Veröffentlichungstag und Bekanntmachung des Hinweises auf die Patenterteilung: **19.08.2009**
(21) Anmeldenummer: 03706577.8
(22) Anmeldetag: 25.02.2003
(51) Int. Cl.: C12Q 1/68

(54) **VERFAHREN ZUR QUALITATIVEN UND QUANTITATIVEN BESTIMMUNG VON GENETISCHEM MATERIAL**
METHOD FOR THE QUALITATIVE AND QUANTITATIVE DETERMINATION OF GENETIC MATERIAL
PROCEDE DE DETERMINATION QUALITATIVE ET QUANTITATIVE DE MATERIAU GENETIQUE

(30) Priorität: 27.02.2002 DE 10208399
(43) Veröffentlichungstag der Anmeldung: 24.11.2004
(73) Patentinhaber: Bezirksverband Pfalz, 67653 Kaiserslautern (DE)
(72) Erfinder: HORMISCH, Diana, 67346 Speyer (DE); STRAUSS, Gerhard, 67346 Speyer (DE)
(74) Vertreter: Dörries, Hans Ulrich
(86) Internationale Anmeldenummer: PCT/EP2003/001921
(87) Internationale Veröffentlichungsnummer: WO 2003/072815

(56) Entgegenhaltungen:
- EP-A- 0 742 286
- WO-A-97/38132
- US-B1- 6 197 515
- ZHAI J ET AL: "DNA based biosensors" BIOTECHNOLOGY ADVANCES, ELSEVIER PUBLISHING, BARKING, GB, Bd. 15, Nr. 1, 1997, Seiten 43-58, XP004059929 ISSN: 0734-9750

## Beschreibung

Die Erfindung betrifft ein Verfahren zur qualitativen und quantitativen Bestimmung von genetischem Material, das neben oder im Gemisch mit anderem genetischen Material vorliegen kann.

### 1. Stand der Technik

Verfahren zum quantitativen Nachweis bestimmter Organismen spielen in verschiedenen Bereichen, z.B. in der Nahrungsmittelindustrie, der Landwirtschaft, der Qualitätskontrolle, im Gesundheitswesen und in der Forschung eine wichtige Rolle. Sie werden z.B. in der Lebens- und Futtermitteldiagnostik zur Bestimmung von pathogenen neben apathogenen Organismen sowie zur Bestimmung des Anteils einzelner tierischer, pflanzlicher oder mikrobieller Komponenten von Lebens- oder Futtermitteln verwendet. In der Biotechnologie werden solche Verfahren beispielsweise zur Bestimmung der Expression von Genen und in der Medizin u.a. zur Bestimmung der Häufigkeit von bestimmten Zellen in Gewebematerial benötigt. In der Mikrobiologie und auf dem Hygienesektor werden ebenfalls Verfahren zur Bestimmung von speziellen Mikroorganismen (Schimmelpilze, Hefen, Bakterien, Viren) neben anderen Mikroorganismen angewandt.

Genetisch veränderten Organismen (GVO) im Nahrungsmittelsektor und in der Landwirtschaft kommt eine immer größere Bedeutung zu. Sie sind wegen der damit möglicherweise verbundenen Risiken Gegenstand gesetzlicher Regelungen, die z.B. eine Deklarierungspflicht bei Überschreiten bestimmter Höchstmengen oder auch Verbote einschließen. Es wird daher ein Verfahren benötigt, nach dem man die Einhaltung dieser Regelungen überwachen, so z.B. den Anteil von transgenem Mais an Mais in Futtermitteln oder den Gehalt von Sojamehl an Mehl von transgenen Sojabohnen bestimmen kann.
Bekannte Verfahren zur quantitativen bzw. semiquantitativen Bestimmung von genetischem Material sind die Mengenabschätzung im Agarose-Gel nach kompetitiver PCR mit anschließender Anfärbung mittels Ethidiumbromid, die Real-time-PCR unter Verwendung von Fluorescenzfarbstoffen sowie im Handel befindliche Testsysteme, die durch Kopplung von PCR und ELISA-Nachweis eine spezifische Amplifizierung von DNA-Sequenzen und deren Nachweis erlauben.

Mit Ausnahme der Real-time-PCR (Hübner, P. et al, J.AOAC Int. 84 (6) (2001), 1855-1864) sind die Ergebnisse dieser Verfahren allenfalls semiquantitativ (Briggs D., International Biotechnology Laboratory 4/2002, 10). Bei der Real-time-PCR unter Verwendung von Fluorescenzfarbstoffen können in Abhängigkeit von der Matrix und der nachzuweisenden Gensequenz Probleme bei der Quantifizierung auftreten (z.B. Quench). Darüber hinaus sind die Untersuchungen auf Grund der zu verwendenden Fluorescenzfarbstoffe kostenintensiv (Pöpping, B, International Laboratory, 31/4 (2001), 23-29).

Bei allen erwähnten Methoden ist eine enzymatische Reaktion zur Quantifizierung des genetischen Materials notwendig. Liegen in dem zu untersuchenden genetischen Material Hemmfaktoren vor, welche die Aktivität des Enzyms beeinträchtigen, kann keine verläßliche quantitative Bestimmung vorgenommen werden.

In WO 97/38132 wird ein Verfahren zur Analyse von Nukleinsäuren beschrieben. Eine der beiden offenbarten Ausführungsformen zielt auf den Nachweis von Abweichungen in der Nukleotidfolge bekannter DNA-Sequenzen (Zielsequenzen) von der üblichen Abfolge. Dazu immobilisiert man vorteilhaft die Zielsequenz auf einer festen Phase, macht sie einzelsträngig und inkubiert sie mit einer Mischung von kurzkettigen Oligonukleotiden, die ganz oder teilweise zu der Zielsequenz komplementär sind. Die feste Phase wird von der flüssigen abgetrennt, in der letzteren werden die nicht hybridisierten Anteile der eingesetzten Nukleotidsonden mit immobilisierten einzelsträngigen DNA-Sequenzen, die zu den Nukleotidsonden komplementär sind, hybridisiert, und die Menge der an diese komplementären, immobilisierten DNA-Sequenzen gebundenen Nukleotidsonden wird bestimmt. Eine erhöhte Konzentration einer oder mehrerer Sonden zeigt eine Abweichung in der Nukleotidfolge der Zielsequenz an.

In einer anderen Ausführungsform eignet sich das Verfahren der WO 97/39132 zur Quantifizierung von amplifizierten Nukleinsäureprodukten. Dazu wird die Zielsequenz zusammen mit einer bekannten Menge einer kompetitiven Nukleinsäure amplifiziert. Das Gemisch der Amplifikate wird auf einer festen Phase immobilisiert, einzelsträngig gemacht und mit (i) einer zu der Zielsequenz komplementären Sonde, (ii) einer zu der kompetitiven Nukleinsäure komplementären Sonde und (iii) wahlweise einer dritten Sonde, die sowohl zu der Zielsequenz, als auch zu der kompetitiven Nukleinsäure komplementär ist, inkubiert. Danach werden die in der Lösung verbliebenen, nicht hybridisierten Sonden bestimmt, wie zuvor beschrieben. Aus den Konzentrationswerten wird die Menge der amplifizierten Zielsequenz und damit auch die Menge der ursprünglich vorhandenen Zielsequenz bestimmt.

### 2. Aufgaben der Erfindung

Eine Aufgabe der Erfindung besteht darin, ein vorteilhaftes Verfahren zur qualitativen und quantitativen Bestimmung von genetischem Material bereitzustellen, das eine charakteristische, bekannte Nukleotidsequenz enthält und neben oder im Gemisch mit anderem genetischen Material vorliegen kann. Eine weitere Aufgabe der Erfindung ist die Bereitstellung eines Verfahrens zur Bestimmung der Konzentration eines genetischen Materials in einer Suspension oder Lösung. Eine andere Aufgabe der Erfindung besteht darin, ein Verfahren bereitzustellen, das die ungenaue und aufwendige Quantifizierung von markierten PCR-Amplifikaten vermeidet und daher eine zuverlässige, schnelle und preisgünstige quantitative Bestimmung ermöglicht. Weitere Aufgaben und Vorteile der Erfindung werden aus der folgenden Beschreibung offenbar werden.

### 3. Kurzbeschreibung der Erfindung

Gegenstand der Erfindung ist ein Verfahren zur qualitativen und quantitativen Bestimmung von genetischem Material, das eine charakteristische, bekannte Nukleotidsequenz (im folgenden "Zielsequenz" genannt) enthält und neben oder im Gemisch mit anderem genetischen Material vorliegt Das Verfahren ist **dadurch gekennzeichnet, daß** man
eine Suspension oder Lösung, die das genetische Material mit der Zielsequenz sowie anderes genetisches Material (im folgenden zusammen "gesamtes genetisches Material" genannt) enthält, mit (1) einem molaren Überschuß einer ersten Oligonukleotidsonde (im folgenden "erste Sonde" genannt) versetzt, deren Nukleotidsequenz zu der Zielsequenz komplementär ist, und (2) mit einem molaren Überschuß einer zweiten Oligonukleotidsonde (im folgenden "zweite Sonde" genannt) versetzt, deren Nukleotidsequenz zu einer Nukleotidsequenz (im folgenden "Kontrollsequenz" genannt) komplementär ist, die in allen Komponenten des gesamten genetischen Materials enthalten ist, auf die sich die Quantifizierung beziehen soll,
das gesamte genetische Material, soweit es doppelsträngig ist, einsträngig macht, die erste Sonde mit der Zielsequenz und die zweite Sonde mit der Kontrollsequenz hybridisiert,
in der Suspension oder Lösung die Konzentrationen der nicht hybridisierten ersten und zweiten Sonde bestimmt und aus den Konzentrationen den Anteil des genetischen Materials mit der Zielsequenz an dem gesamten genetischen Material, auf das sich die Quantifizierung beziehen soll, ermittelt.

Das erfindungsgemäße Verfahren arbeitet in einer ersten Ausführungsform mit zwei Sonden und ergibt das Mengenverhältnis des zu bestimmenden genetischen Materials und eines neben oder im Gemisch damit vorliegenden anderen genetischen Materials, z.B. als Gewichtsprozent in bezug auf die Summe der beiden genetischen Materialien.

Eine zweite Ausführungsform des Verfahrens arbeitet mit nur einer Sonde und dient zur Bestimmung der Konzentration von genetischem Material in einer Suspension oder Lösung, z.B. als Keimzahl pro Volumeneinheit. Diese Ausführungsform ist dementsprechend ein Verfahren zur quantitativen Bestimmung der Konzentration von genetischem Material, das eine charakteristische, bekannte Nukleotidsequenz (im folgenden "Zielsequenz" genannt) enthält und neben oder im Gemisch mit anderem genetischen Material vorliegen kann. Das Verfahren ist **dadurch gekennzeichnet, daß** man
eine Suspension, die das genetische Material mit der Zielsequenz sowie gegebenenfalls anderes genetisches Material enthält, mit einem molaren Überschuß nur einer Oligonukleotidsonde (der "ersten Oligonukleotidsonde" der zuvor beschriebenen ersten Ausführungsform) versetzt, deren Nukleotidsequenz zu der Zielsequenz komplementär ist,
das genetische Material, soweit es doppelsträngig ist, einsträngig macht, die Sonde mit der Zielsequenz hybridisiert,
in der Suspension oder Lösung die Konzentration der nicht hybridisierten Sonde bestimmt und aus dieser Konzentration die Konzentration des genetischen Materials in der Suspension ermittelt.

### 4. Merkmale, Vorteile und Anwendungsmöglichkeiten des Verfahrens

Beide beschriebenen Ausführungsformen des erfindungsgemäßen Verfahrens bestimmen die Menge(n) bzw. Konzentration(en) der nicht hybridisierten Sonde(n). Die Differenz aus den Mengen der eingesetzten und der nicht hybridisierten Sonde entspricht dem jeweils zu bestimmenden genetischen Material.

Das Verfahren nach der Erfindung ermöglicht sowohl einen qualitativen Nachweis als auch eine quantitative Bestimmung von genetischem Material. Es ist in seiner ersten Ausführungsform, ebenso wie die zweite Ausführungsform des Verfahrens nach WO 97/38132, auf die Quantifizierung von genetischem Material gerichtet. Im Gegensatz zu dem bekannten Verfahren muß das genetische nicht durch Amplifizierung hergestellt werden. Ein weiterer Unterschied besteht darin, daß bei dem bekannten Verfahren eine kompetitive Nukleinsäure als Hilfsstoff eingesetzt werden muß, während das Verfahren nach der Erfindung ohne einen solchen Hilfsstoff auskommt. Weiterhin wird erfindungsgemäß das Mengenverhältnis zweier genetischer Materialien mittels zweier Sonden bestimmt, von denen die eine mit der Zielsequenz und die andere mit einer Kontrollsequenz hybridisiert wird, die in beiden genetischen Materialien enthalten ist. Das bekannte Verfahren arbeitet dagegen mit (i) einer zu der Zielsequenz komplementären Sonde, (ii) einer zu der kompetitiven Nucleinsäure komplementären Sonde und (iii) nur wahlweise einer dritten Sonde, die sowohl zu der Zielsequenz, als auch zu der kompetitiven Nucleinsäure komplementär ist. Es verwendet also entweder keine Sonde, die sowohl zur Zielsequenz als auch zur kompetitiven Sequenz komplementär ist, oder aber erfordert zusätzlich zu einer solchen Sonde zwei weitere Sonden.

Die zweite Ausführungsform des erfindungsgemäßen Verfahrens zielt auf die Bestimmung der Konzentration eines genetischen Materials in einer Suspension oder Lösung und unterscheidet sich schon hierdurch von der ersten Ausführungsform des Verfahrens nach WO 97/38132, die die Aufklärung von Abweichungen in der Nukleotidfolge von genetischem Material bezweckt. Dementsprechend findet sich in der Druckschrift nicht die Lehre, aus den Konzentrationen der nicht hybridisierten Sonden einen Schluß auf die Konzentration des genetischen Materials in der Ausgangssuspension oder -lösung zu ziehen.

Auch gegenüber den anderen zuvor beschriebenen Verfahren nach dem Stand der Technik bietet das erfindungsgemäße Verfahren eine Reihe von Vorteilen. Eine markierungsfreie Detektion zur Bestimmung der Sondenkonzentrationen nach der Hybridisierung durch Verwendung von Biosensoren vermeidet die aufwendige und ungenaue Quantifizierung mittels markierter PCR-Amplifikate. Durch Miniaturisierung und Automatisierung werden Simultanmessungen möglich. Weitere Vorteile sind die leichte Regenerierbarkeit der Biosensoren, geringere Proben- und Reagenzienvolumina und eine deutliche Arbeitserleichterung. Die hochaffinen Wechselwirkungen zwischen den Biomolekülen erlauben quantitative Messungen mit weniger vorgereinigten genetischen Materialien. Kostspielige Reagenzien, wie für den zuvor erwähnten Fluoreszenznachweis erforderlich, werden nicht benötigt. Weiterhin ist der Zeitbedarf für das Verfahren nach der Erfindung geringer als bei bekannten Verfahren.

Erfindungsgemäß sind z.B. Rückschlüsse auf Gen-, Transkript-, Expressions-, Zell- oder Organismushäufigkeiten und Organismusverteilungen oder Produktzusammensetzungen möglich. Dabei kann z.B. DNA neben DNA, RNA neben RNA und DNA neben RNA bestimmt werden. Im einzelnen eignet sich das Verfahren nach der Erfindung in seinen beiden Ausführungsformen hervorragend z.B. zur Lösung der unter vorstehender Ziffer 1 beschriebenen analytischen Aufgaben. Es löst insbesondere z.B. die folgenden Probleme:
□ Identifizierung und Quantifizierung von DNA-Fragmenten nach gelelektrophoretischer Auftrennung eines DNA-Gemisches. In diesem Fall liegt kein anderes genetisches Material neben dem genetischen Material mit der Zielsequenz vor.
□ Konzentrationskontrolle von Zellen in biotechnologischen Verfahren. Hier kann anderes genetisches Material neben dem Material mit der Zielsequenz vorliegen.
□ Bestimmung der Konzentration von DNA- oder RNA-haltigem biologischem Material im Blut oder in anderen Flüssigkeiten.
□ Bestimmung des Anteils einer bestimmten Bakterienspezies an einer Bakterienpopulation, z.B. einer bestimmten Milchsäurebakterienspezies an der gesamten Milchsäurebakterienpopulation. Die Zielsequenz ist DNA. Wenn nur die Anzahl der bestimmten Milchsäurebakterien pro Volumeneinheit bestimmt werden soll, ist nur die erste Sonde erforderlich. Soll der Anteil der bestimmten Milchsäurebakterien an der gesamten Milchsäurebakterienpopulation bestimmt werden, so bedarf es auch der zweiten Sonde. Die Kontrollsequenz ist dann ebenfalls DNA. Das gesamte genetische Material stammt aus genetisch verschiedenen, wenn auch weitgehend identischen Organismen mit gemeinsamer Kontrollsequenz.
□ Bestimmung des Gehalts von Sojamehl an GVO-Soja oder von Maismehl an GVO-Mais. Die Zielsequenz und die Kontrollsequenz sind DNA, und das gesamte genetische Material stammt wiederum aus genetisch verschiedenen, wenn auch weitgehend identischen Organismen.
□ Bei der Untersuchung der Plasmidhäufigkeit in einer Bakterienzelle ist die Zielsequenz Plasmid-codiert und die Kontrollsequenz chromosomal codiert, und man bestimmt zwei DNA-Sequenzen, die aus dem selben Organismus stammen.
□ Wenn die Zahl der RNA-Kopien eines Gens im Zellplasma bestimmt wird, um Aufschluß darüber zu erhalten, wie aktiv das Gen zu dem betreffenden Zeitpunkt oder unter den herrschenden Bedingungen ist, werden RNA und DNA ins Verhältnis gesetzt, und das genetische Material stammt ebenfalls aus nur einem Organismus.
□ Bei der Bestimmung des Mengenverhältnisses von RNA-Kopien von Genen wird RNA mit RNA verglichen, und das genetische Material stammt wiederum aus nur einem Organismus.

### 5. Detaillierte Beschreibung der Erfindung

Das erfindungsgemäße Verfahren wird zunächst mit Bezug auf die erste der beiden Ausführungsformen beschrieben.

### 5.1 Das genetische Material

Der Terminus genetisches Material wird hierin synonym mit Nukleinsäure gebraucht. Das genetische Material kann einzelsträngig oder doppelsträngig sein. Es kann DNA, RNA oder PNA sein oder andere modifizierte Basensequenzen enthalten und stammt in der Regel aus biologischem Material, das aus nur einem Organismus bestehen oder mehrere, verschiedene Organismen enthalten kann. Das biologische Material schließt z.B. Viren, Bakterien, Pilze, Hefen, Pflanzen, Tiere oder Teile davon oder deren Produkte oder Verarbeitungsprodukte ein. Das gesamte genetische Material, von dem das Verfahren nach der Erfindung ausgeht, kann in üblicher Weise aus dem entsprechenden biologischen Material gewonnen werden. Je nach Zielsetzung werden geeignete bekannte Verfahren zur Isolierung oder Anreicherung der Zielsequenz und/oder_der Kontrollsequenz eingesetzt.

Die Zielsequenz und die Kontrollsequenz sind Nukleotidsequenzen mit bekannter Basenabfolge. Sie können ein Gen, Plasmid, RNA- oder PNA-Molekül oder eine andere modifizierte Basensequenz sein. Auch Fragmente dieser Konstrukte können die Zielsequenz bzw. Kontrollsequenz sein.

Es ist möglich, daß sich die Quantifizierung nur auf einen Teil des gesamten genetischen Materials beziehen soll. Wenn beispielsweise der Anteil von GVO-Soja in einem Futtermehl bestimmt wird, kann man diesen Anteil auf das gesamte Futtermehl oder, was häufig erwünscht ist, auf den Anteil an Sojamehl im Futtermehl beziehen. In diesem Fall wählt man eine Kontrollsequenz, die nur in GVO- und natürlichem Mais vorkommt, und eine dementsprechende zweite Sonde.

### 5.2 Hybridisierung der Sonden mit Zielsequenz und Kontrollsequenz

Das Verfahren nach der Erfindung geht von einer Pufferlösung aus, die das gesamte genetische Material suspendiert oder gelöst enthält. Die Suspension oder Lösung wird mit einem molaren Überschuß der ersten und der zweiten Sonde versetzt. Molarer Überschuß bedeutet, daß auf jede der Zielsequenzen bzw. Kontrollsequenzen im gesamten genetischen Material mindestens ein Molekül Sonde entfällt. Man kann die für einen Überschuß erforderlichen Sondenmengen durch Vorversuche bestimmen, wobei man mit geringen Mengen der Sonden beginnt. Ein Unterschuß macht sich dadurch kenntlich, daß die Sonde nach der Hybridisierung nicht mehr nachweisbar ist. Man steigert die Konzentrationen, bis man ein deutlich meßbares Signal erhält. Die optimale Höhe des Überschusses der Sonden hängt maßgeblich von der Zahl der in der Suspension oder Lösung vorhandenen Ziel- bzw. Kontrollsequenzen ab und muß von Fall zu Fall ermittelt werden.

Die beiden Sonden enthalten eine einzelsträngige Oligonukleotidsequenz (z.B. aus DNA, RNA, PNA oder anderen modifizierten Basensequenzen). Sie umfassen zweckmäßig 6 bis 70 Nukleotide, insbesondere 13 bis 25 Nukleotide und sind zu der gesamten oder zu einem Teil der Zielsequenz bzw. der Kontrollsequenz komplementär. Die Abfolgen der Nukleotide der Zielsequenz und der Kontrollsequenz müssen bekannt sein, damit die Sonden nach üblichen Verfahren in komplementärer Basenfolge hergestellt werden können. Sonden mit der gewünschten Basenfolge sind im Handel erhältlich. Sie werden, wie das genetische Material, vorteilhaft in Pufferlösung suspendiert oder gelöst angewandt.

Soweit das gesamte genetische Material doppelsträngig ist, muß es vor der Hybridisierung einzelsträngig gemacht werden. Dazu erhitzt man die Lösung zweckmäßig 5 bis 20 Minuten auf eine Temperatur von 95 bis 100°C und läßt sie DNA langsam auf Raumtemperatur abkühlen, wobei die Sonden mit der Zielsequenz bzw. Kontrollsequenz hybridisieren. Die Hybridisierung ist je nach den Hybridisierungsbedingungen und der Sondenbeschaffenheit (Nukleotidabfolge und Sondenlänge) im allgemeinen nach 1 bis 120 min beendet. Nach der Hybridisierung bestimmt man die Konzentration der nicht hybridisierten Sonden.

### 5.3 Bestimmung der Konzentrationen der beiden nicht hybridisierten Sonden

Zur Bestimmung der Konzentrationen der nicht hybridisierten Sondenmoleküle bedient man sich vorteilhaft eines der verschiedenen markierungsfreien Detektionsverfahren, die speziell für die Analyse von biomolekularen Interaktionen entwickelt wurden. Ein Überblick über die verschiedenen Verfahren der Bioaffinitätsanalytik findet sich bei Bier, F.F. et al in Biosensoric 1, Fundamental Aspects (1997), herausgegeben von Scheller, F.W., Schubert, F. und Fedrowitz, J., Birkhäuser Verlag, Basel, Schweiz. Ein Biosensor ist eine Anordnung, in der ein biologisch sensitives Element mit einem physikalischen Wandler (Transducer) gekoppelt ist. Dabei wird die hochspezifische Reaktion der Sondenmoleküle mit spezifischen Biomolekülen mittels des Transducers in ein meßbares Signal übersetzt. Elektrochemische Verfahren und Transducer werden von Ullman, M., Synthese von Peptiden zur Verwendung in einem Biosensor (1998), Reihe Chemie, ad fontes-Verlag, ISBN 3-932681-08-8, piezoelektrische bzw. massensensitive Verfahren von Liebau, M., Resonante Quarzsensoren, Dissertation Universität Halle-Wittenberg (1998) und optische von Brecht, A. und Gauglitz, G., Optical probes and transducers, Biosens&Bioelectron, 10 (1995) 923-936 beschrieben. Bei all diesen Methoden liegt der Analyt, also die Sondenmoleküle, in Lösung vor, während der andere Reaktionspartner als Ligand auf einer Oberfläche immobilisiert ist.

Sehr empfindlich sind optische Verfahren, die daher bevorzugt werden. Eines dieser Verfahren bedient sich des Phänomens der Oberflächenplasmonresonanz (Surface Plasmon Resonance; SPR). Das Verfahren ist ausführlich in der Publikation von K.Nagata und H.Handa, Real-time Analysis of Biomolecular Interactions: Applications of BIACORE, Springer-Verlag Tokyo Berlin Heidelberg New York, ISBN 4-431-70289-X, insbesondere auf den Seiten 1 bis 83, beschrieben. Grundprinzip aller SPR-Sensoren ist das Phänomen der optischen Anregung der Schwingung von Elektronen in einem dünnen Metallfilm (Kretzmann, E., und Raether, H., Radiative decay of non-radiative surface plasmons by light, Z. Naturforsch., Vol 23a (1968), 2135). Die durch Wechselwirkung mit den Photonen entstandenen Schwingungen, sogenannte Polaritone oder Plasmone, breiten sich über einige Mikrometer wellenförmig in der Metallschicht aus. Die Schwingungen sind verbunden mit einem evanescenten Feld, das über die Phasengrenze in die Umgebung hineinreicht. Über ein Prisma, das von der Rückseite des Sensorchips gegen ein Glassubstrat drückt, wird monochromatisches Licht eingestrahlt und anschließend das Winkelspektrum des reflektierten Lichts ausgelesen. Da Licht einer bestimmten Wellenlänge und eines bestimmten Einstrahlwinkels zur Anregung des SPR-Effekts absorbiert wird, erscheint in der Reflexion ein Intensitätsminimum. Kommt es nun auf der Metalloberfläche des Sensorchips zu geringen Änderungen der Massebeladung, z.B. durch Anlagerung von Proteinen oder Nukleinsäuren, so wechselwirken die Moleküle mit dem elektromagnetischen evanescenten Feld. Ergebnis dieser Wechselwirkung ist eine detektierbare Veränderung des Intensitätsminimums im Reflexionswinkelspektrum, die mit der Massezunahme korreliert (Kuhlmeier, D., Nachweis von Nukleinsäure-Wechselwirkungen mit optischen Biosensoren, Dissertation TH Braunschweig (2000)). So können Wechselwirkungen von Molekülen mit dem evanescenten Feld höchst sensitiv detektiert werden.

Verschiedene Modelle von Meßgeräten nach dem SPR- Verfahren werden, einschließlich der Auswertungssoftware, von der Firma Biacore AB, Rapsgatan 7, S-754 50 Uppsala, Schweden, vertrieben. Das Biacore-Meßsystem gibt das Sensorsignal in sogenannten Resonance Units (RU) an, wobei willkürlich festgesetzt wurde, daß 1.000 RU einer Anlagerung von 1 ng/mm² entspricht (Stenberg, E. et al, Quantitative determination of surface concentration, J.Coll.Interf.Sci., 143, 513-526 (1991)). 1.000 RU entsprechen andererseits einer Ablenkung des Reflexionswinkels um 0,1°. Dargestellt werden die Daten in einem Diagramm mit Ordinate (RU) und Abszisse (Zeit).

Zur Detektion der jeweiligen nicht hybridisierten Sonde wird auf dem Sensorchip ein entsprechender Ligand immobilisiert. Hierbei handelt es sich, je nach Beschaffenheit der Ziel- bzw. Kontrollsequenz, um DNA-, RNA-, PNA- oder ähnliche Basensequenzen, die zu den Basensequenzen der Sonden komplementär sind (Kilsa Jensen et al, Kinetics for Hybridization of Peptide Nucleic Acids, Biochemistry, 36, 5072-5077 (1997)) und zudem noch funktionelle Gruppen für eine auf kovalenter Bindung oder auf physikalischen Wechselwirkungen beruhende Immobilisierung auf dem Sensorchip tragen. Sie hybridisieren mit den Sondenmolekülen aufgrund spezifischer Basenpaarung über Wasserstoffbrückenbindungen. Eine für die Immobilisierung der Ligandenmoleküle geeignete funktionelle Gruppe ist z.B. Biotin, sofern Streptavidin auf dem Sensorchip dotiert ist. Solche Sensorchips vertreibt z.B. die zuvor erwähnte Firma Biacore AB. Biotingruppen enthaltende Oligonukleotide mit den gewünschten Nukleotidsequenzen sind im Handel erhältlich.

Zur Messung der Konzentration der nicht hybridisierten Sondenmoleküle strömt die Suspension mit einer definierten Temperatur von beispielsweise 25,0°C und mit einer definierten Geschwindigkeit beispielsweise von 25 µl/min an dem Sensorchip bzw. nacheinander oder in parallelen Strömen an den Sensorchips vorbei, wobei jeder Sensorchip bzw. jede Meßstelle auf einem Sensorchip mit einem Oligonukleotid beladen ist, das zu der zu messenden Sonde komplementär ist.

Nach der Messung werden die Sensorchips regeneriert und für eine neue Messung vorbereitet, indem man die gebundenen Sondenmoleküle mit einer geeigneten Lösung (zweckmäßig eine 50 mM NaOH-Lösung; 10 sec) von den immobilisierten Oligonukleotiden unter Bedingungen ablöst, die diese auf dem Sensorchip belassen.

### 5.4 Ermittlung des Anteils des genetischen Materials mit der Zielsequenz an dem gesamten genetischen Material

Zur Ermittlung des Anteils des genetischen Materials, das die Zielsequenz enthält, am gesamten oder dem Teil des genetischen Materials, auf das sich die Quantifizierung beziehen soll, muß die Meßanordnung geeicht werden, was zweckmäßig vor der Messung geschieht. Dazu läßt man Lösungen mit bekannten, variierenden Konzentrationen an erster bzw. zweiter Sonde, die kein genetisches Material oder das gesamte genetische Material ohne Hybridisierungsschritt enthalten, unter Meßbedingungen (Temperatur, Strömungsgeschwindigkeit an den Sensorchips vorbeiströmen und mißt jeweils das SPR-Signal. Der Zusammenhang zwischen der Konzentration der Sondenmoleküle und der Stärke des SPR-Signals läßt sich in einer Eichkurve graphisch darstellen.

Die Messungen der Konzentration der ersten Sonde nach der Hybridisierung mit der Zielsequenz und der Konzentration der zweiten Sonde nach der Hybridisierung mit der Kontrollsequenz ergeben zwei Werte. Wenn die Konzentration der ersten Sonde nach der Hybridisierung im Vergleich zu der Konzentration ohne Hybridisierung unverändert geblieben ist, war kein genetisches Material mit der Zielsequenz in dem gesamten genetischen Material vorhanden. Wenn die Konzentration der ersten Sonde nach der Hybridisierung im Vergleich zu der Konzentration ohne Hybridisierung vermindert ist, so ist damit genetisches Material mit der Zielsequenz qualitativ nachgewiesen. Die quantitative Bestimmung, also der Anteil des genetischen Materials mit der Zielsequenz am gesamten genetischen Material mit der Kontrollsequenz, ergibt sich aus dem Vergleich der gemessenen Konzentrationen der ersten und der zweiten Sonde, jeweils mit und ohne Hybridisierung.

### 5.5 Das Verfahren in seiner zweiten Ausführungsform

Die Beschreibung des Verfahrens nach der ersten Ausführungsform gilt mutatis mutandis auch für die zweite Ausführungsform. Es gibt jedoch keine Kontrollsequenz, keine zweite Oligonukleotidsonde und keinen Sensorchip, auf dem ein zu der zweiten Oligonukleotidsonde komplementärer Ligand immobilisiert ist. Beispielsweise kann die Bestimmung der Konzentration von Viren im Blut so erfolgen, daß man eine virale Zielsequenz auswählt, ein definiertes Volumen viröses Blut unter Hybridiserungsbedingungen mit einem definierten molaren Überschuß einer zu der Zielsequenz komplementären Oligonukleotidsonde in Berührung bringt ("Sondenhybridisierung"), eine zu dieser Oligonukleotidsonde komplementäre Oligonukleotidsequenz als Ligand immobilisiert und die Suspension oder Lösung, die die hybridisierte Zielsequenz und die nicht hybridisierten Nukleotidsonden enthält, an den immobilisierten Oligonukleotidsequenzen vorbeiströmen läßt ("Ligandenhybridisierung"). Das Detektorsignal wird anhand einer zuvor ermittelten Eichkurve einer bestimmten molaren Menge an Oligonukleotidsonde zugeordnet und ergibt durch Differenzbildung die durch die Sondenhybridisierung verbrauchte molare Menge an Oligonukleotidsonde. Diese ist der molaren Menge an viraler Zielsequenz und damit auch an Viren äquivalent. Die Division durch das definierte Volumen ergibt deren Konzentration.

### 6. Spezielle Detektionsmethoden

6.1 Hybridisierung der Sonden mit dem genetischen Material
   In einer Variante der ersten Ausführungsform des Verfahrens nach der Erfindung wird die Hybridisierung der ersten und der zweiten Sonde in Einzelschritten mit Teilmengen desselben genetischen Materials durchgeführt. Alternativ kann man die erste und die zweite Sonde mit dem genetischen Material in ein und demselben Reaktionsansatz hybridisieren und anschließend die überschüssigen, nicht gebundenen Sondenmoleküle bestimmen.
6.2 Rezeptor/Zielmolekül-Reaktionen als sionalgebende Bioreaktionen
   Zuvor wurde beschrieben, daß die nicht hybridisierten Oligonukleotidsonden (als Zielmoleküle) durch immobilisierte Oligonukleotide (als Rezeptoren) aufgrund spezifischer Basenpaarung gebunden werden. Dieses Bindungsprinzip kann auf verschiedene Weise ersetzt werden:
   6.2.1 So kann statt eines Oligonukleotids ein immobilisierter Antikörper (als Rezeptor) gegen ein Antigen (als Zielmolekül) verwendet werden, das seinerseits an die Sondenmoleküle gebunden ist. Beispielsweise kann man auf dem Sensorchip Anti-Dig (Dig = Digoxigenin) immobilisieren und die Sonde mit dem Antigen Dig verknüpfen. Die Sondenmoleküle werden dann mittels einer Antigen/Antikörper-Reaktion mit dem immobilisierten Antikörper aus der Lösung entfernt und signalgebend an der Grenzfläche angereichert.
   6.2.2 Weiterhin kann man statt eines Oligonukleotids als Rezeptor ein Antigen (z.B. Dig) auf dem Sensorchip immobilisieren und die Sonde mit dem gleichen Antigen verknüpfen. Nach der Hybridisierung werden die nicht hybridisierten Dig-modifizierten Sonden moleküle mit einem definierten Überschuß an Antikörper (Anti-Dig) versetzt. Der dabei nicht gebundene überschüssige Antikörper geht als Zielmolekül eine Antigen/Antikörper-Reaktion mit dem immobilisierten Antigen-Rezeptor ein, wodurch wiederum ein Biosignal erzeugt wird.
6.3 Verfahren mit vorgeschalteter PCR
   Wenn im Einzelfall für eine hinreichend genaue Bestimmung eine zu geringe Menge gesamtes genetisches Material zur Verfügung steht oder das gesamte genetische Material nur sehr geringe Mengen genetisches Material mit der Zielsequenz enthält, kann man deren Menge, z.B. mittels PCR, vergrößern. Die Bestimmung der restlichen Sonden nach der Hybridisierung mittels SPR (und damit die Bestimmung der durch Hybridisierung verbrauchten Sonden) ist erheblich genauer als die Bestimmung von Amplifikat auf dem Gel nach der Fluorescenzmethode.
6.4 Erhöhung der Sensitivität bzw. Senkung der Nachweisgrenze
   Die Stärke des Bio-Signals ist, wie zuvor erläutert, abhängig von der Konzentration der Sondenmoleküle an der Grenzfläche. Sie hängt aber auch, bei gleicher Konzentration, von deren Molmassen ab. Durch Erhöhung der Molmassen der Sondenmoleküle läßt sich das Biosensor-Signal und damit die Empfindlichkeit des Verfahrens erheblich steigern:
   6.4.1 Beispielsweise läßt sich die Molmasse von Antigen-modifizierten Sonden (siehe 6.2.2) steigern, wenn man einen gegen das Antigen gerichteten Antikörper auf sie einwirken läßt und gegebenenfalls das so vergrößerte Molekül mit einem weiteren, gegen den ersten Antikörper gerichteten zweiten Antikörper weiter vergrößert. Beispielsweise kann die Molmasse einer Dig-modifizierten Sonde mit Anti-Dig und weite mit Anti-Anti-Dig vergrößert werden.
   6.4.2 Die Molmassen von gegebenenfalls Antigen-modifizierten Sonden lassen sich vergrößern, wenn man die Zahl der Nukleotide über das für eine sichere Erkennung der Zielsequenz wünschenswerte Maß von etwa 20 hinaus auf bis zu etwa 70 vergrößert.
6.5 Universalchip-Strategie für verschiedene Sonden
   6.5.1 Wenn man mit dem Nachweissystem Antigen/Antikörper-Reaktion gemäß 6.2.1, 6.2.2, 6.4.1 oder 6.4.2 arbeitet und sowohl die erste als auch die zweite Sonde mit dem gleichen Antigen verknüpft, kann man den selben Sensorchip für beide Messungen verwenden.
   6.5.2 Das Nachweissystem, das auf der spezifischen Basenpaarung beruht, kann generalisiert werden, indem man zunächst einen (immobilisierenden) Vorhybridisierungsschritt mit einem Bindungsvermittler durchführt, der eine erste, zu der auf dem Sensorchip immobilisierten Nukleotidsequenz komplementäre Nukleotidsequenz und auch eine zweite, zu der Zielsequenz bzw. zu der Kontrollsequenz komplementäre Nukleotidsequenz aufweist. Man verwendet erste und zweite Sonden, die eine zu der nach der Vorhybridisierung noch freien zweiten Nukleotidsequenz des Bindungsvermittlers komplementäre Nukleotidsequenz enthalten, und hybridisiert die überschüssige erste und zweite Sonde mit dem immobilisierten Bindungsvermittler. Der erste Hybridisierungsschritt gibt Aufschluß über die Qualität des Sensorchips hinsichtlich der Zahl der zu besetzenden Stellen. Mit stringenten Waschschritten können die Sondenmoleküle und der Bindungsvermittler von dem immobilisierten Rezeptor abgelöst werden, so daß der Sensorchip mit einem anderen Bindungsvermittler andere Sondenmoleküle binden kann.
6.6 Kontrollbestimmungen
   Zur Überprüfung der Ergebnisse mit einer bestimmten ersten und einer bestimmten zweiten Sonde kann man zusätzlich alternative erste und/oder zweite Sonden einsetzen, die mit anderen Basenfolgen der Zielsequenz bzw. der Kontrollsequenz hybridisieren. Die alternativen Sonden können mit den zu überprüfenden Sonden mehr oder minder überlappen oder aber eine komplett andere Basenfolge aufweisen.

Geeignete Antigene und Antikörper sowie mit Antigenen oder Antikörpern verknüpfte Sonden mit gewünschter Nukleotidsequenz, wie sie in den vorstehenden Abschnitten dieser Ziffer 6 erwähnt sind, können nach üblichen Methoden hergestellt werden, sind im Handel erhältlich.

Die unter der Ziffer 6 aufgeführten Maßnahmen können alternativ und gegebenenfalls kumulativ getroffen werden.

### Beispiel

### Bestimmung von GVO-Mais in Maismehl

### Vorbereitung des genetischen Materials

Es wurden 2 g Maismehl eingesetzt, in dem der Anteil an GVO-Mais quantifiziert werden sollte. Die Extraktion der DNA erfolgte mit Standardmethoden (Mühlhardt, C., Der Experimentator: Molekularbiologie, 2. Auflage (2000), S. 11-37, Spektrum Akademischer Verlag). Da die Zielsequenz nur einen geringen Anteil am gesamten genetischen Material ausmachte, wurde sie zur Anreicherung mittels PCR amplifiziert. In der folgenden Tabelle 1 sind die wesentlichen Daten zur Amplifikation eingesetzten Primer zusammengefaßt.

**Tabelle 1 Eingesetzte PCR-Primer zur Amplifikation der Zielsequenzen**

| Bezeichnung | | Detektionsziel | Basenfolge der PCR-Primer | Länge |
|---|---|---|---|---|
| 1 | 35S-F | GVO- CaMV Promotor Kontrollelement 35 S | 5'-CCTACAAATGCCATCATTGCG-3' | 21-mer |
| 2 | 35S-R | | 5'-GGGTCTTGCGAAGGATAGTG-3' | 20-mer |
| 3 | Ivr-1 | Invertase-Gen Zea mays | 5'-CCGCTGTATCACAAGGGCTGGTACC-3' | 25-mer |
| 4 | Ivr-2 | | 5'-GGAGCCCGTGTAGAGCATGACGATC- 3' | 25-mer |

Es wurden 35 Zyklen eines Standard-PCR-Programms mit Taq-Polymerase von Quiagen, D-40721 Hilden, BR Deutschland, durchgeführt (Mühlhardt, a.a.O., Seiten 67-81). Mit einer Agarose-Gelelektrophorese (Mühlhardt, a.a.O., Seiten 46-51) wurde das Vorhandensein des Amplifikats überprüft. Es waren keine unspezifischen PCR-Produkte nachweisbar.

### Hybridisierungreaktion der Sonden mit Ziel- und Kontrollsequenz

Eine Oligonukleotidsequenz (= erste Sonde) wurde aus der Nukleotidfolge des CaMV-Promotors (Gen bank accesion number V00141) abgeleitet (Franck, A. et al, Nucleotide sequence of cauliflower mosaic virus DNA, Cell 21 (1980), 285-294). Sie weist eine komplementäre Basenfolge zu einer Zielregion innerhalb der Zielsequenz CaMV35S auf (siehe Tab. 2) und erlaubt einen hochspezifischen Nachweis der Zielregion.

Die erste Sonde wurde in einem Überschuß zur erwarteten Konzentration der Zielsequenz zugegeben:

| | |
|---|---|
| Ansatz: | 15µl Lösung des PCR-Ansatzes, im Verhältnis 1:10 mit 0,5 M NaCl HBS-EP Puffer verdünnt, |
| | 20µl erste Sonde (2mM im Gesamtansatz) |
| | 35µl 0,5 M NaCl HBS-EP Puffer |

Der Ansatz wurde 10 min bei 100°C inkubiert und anschließend auf Raumtemperatur abgekühlt.

Eine weitere Oligonukleotid-Sequenz (= zweite Sonde) wurde aus der Sequenz des Invertase-Gens aus Zea mays (GenBank accession number U16123) abgeleitet (Xu, J. et al, Plant Physiol. 108(3), 1293-1294). Sie weist eine komplementäre Basenfolge zu einer Zielregion innerhalb der Mais-typischen Kontrollsequenz Invertase auf (siehe Tabelle 2) und erlaubt einen hochspezifischen Nachweis der Kontrollsequenz. Diese zweite Sonde wurde mit dem folgenden Versuchsansatz hybridisiert, wie für die erste Sonde beschrieben:

| | |
|---|---|
| Ansatz: | 15µl Lösung des PCR-Ansatzes, im Verhältnis 1:10 mit 0,5 M NaCl HBS-EP Puffer verdünnt, |
| | 20µl zweite Sonde (4mM im Gesamtansatz) |
| | 35µl 0,5 M NaCl HBS-EP Puffer |

### Herstellung der spezifischen Sensoroberflächen

Die Messungen wurden mit einem Biacore Q von Biacore AB, a.a.O., durchgeführt. Es wurde ein Sensorchip eingesetzt, der mit Streptavidin vorbeschichtet war (Biochip SA; Biacore AB, a.a.O.). Auf der Streptavidin-Oberfläche wurde jeweils ein Biotinmarkiertes, zu den beiden Sonden komplementäres Oligonukleotid (MWG-Biotech AG, D-85560 Ebersberg) als Ligand immobilisiert (Wood, S.J., Microchemical Journal 47 (1993), 330-337). In der folgenden Tabelle 2 sind die wesentlichen Daten der verwendeten Liganden aufgeführt.

**Tabelle 2 Eingesetzte Oligonukleotid-Sequenzen zur Detektion der Reduktion der ersten und der zweiten Sonde**

| Bezeichnung | Funktion | Detektionsziel | Länge | Basensequenz |
|---|---|---|---|---|
| 35S-Sondekompl | Ligand am Biochip immobilisiert mit 5' Biotinylierung (in der Basenfolge komplementär zur 1. Sonde) | GVO-Element 35S | 21-mehr | 5'Biotin-AAG AAA AAG GTG CTA CGA GGA -3' |
| 35S-Sonde | Analyt (= 1. Sonde) | | 21-mer | 5'-TCC TCG TAG CAC CTT TTT CTT-3' |
| Ivr-Sondekompl | Ligand am Biochip immobilisiert mit 5' Biotinylierung (in der Basenfolge komplementär zur 2. Sonde) | Bezugsgen Invertase-Gen | 20-mer | 5'Biotin-CAA CCG CAG CAT GCC CAC TA-3' |
| Ivr-Sonde | Analyt (= 2. Sonde) | | 20-mer | 5'-TAG TGG GCA TGC TGC GGT TG-3' |

Zur Immobilisierung des jeweiligen Liganden wurde ein 0,5 M NaCl HBS-EP Puffer (Biacore AB, a.a.O.) als Laufpuffer eingesetzt. Die Fließgeschwindigkeit betrug 30 µl/min. Nach Konditionierung des Sensorchips mit drei einminütigen Injektionen von 1 M NaCl in 50 mM NaOH-Lösung wurden jeweils 60 µg/ml biotinylierte Oligonukleotide injiziert und auf der Oberfläche des Sensorchips immobilisiert.

### Bestimmung der nicht hybridisierten ersten Sonde

Die Reaktion der ersten Sonde mit dem immobilisierten Liganden 35S-Sonde-kompl. wurde kalibriert. Die Fließgeschwindigkeit des Laufpuffers (siehe oben) betrug 25 µl/min. Die Signaldifferenz des Sensorchips, gemessen in resonance units (RU), zwischen der detektierten Basislinie (nur Puffer) und dem erreichten Plateau nach der Probeninjektion wurde zur Bestimmung der Menge an ungebundener erster Sonde verwertet (Wood, a.a.O). Bei der Injektion von 30 µl des Probenansatzes ohne Hybridisierungsreaktion betrug die Signaldifferenz 685,2 RU, entsprechend einer Konzentration an erster Sonde von 1,97 mM. Bei der Injektion von 30 µl des Probenansatzes nach der Hybridisierungsreaktion betrug die Signaldifferenz 442 RU, entsprechend einer Konzentration von 1,16 mM. Es hatten folglich 1,97 mM minus 1,16 mM = 0,81 mM erste Sonde mit der Zielsequenz hybridisiert, das entspricht 0,81 mM Zielsequenz.

Im Anschluß an die Hybridisierungsreaktion auf dem Sensorchip wurde durch Injektion von 15 µl 1 M NaCl in 50 mM NaOH-Lösung die Chipoberfläche von hybridisierten Sondenmolekülen befreit und anschließend mit Laufpuffer neutralisiert.

### Bestimmung der nicht hybridisierten zweiten Sonde

Die Bestimmung erfolgte analog der Bestimmung der nicht hybridisierten ersten Sonde. Das gesamte genetische Material wurde erhalten, indem genetisches Material von 2 g Maismehl unter Verwendung der Primer Ivr-1 und Ivr-2 gemäß Tabelle 1 durch PCR in 35 Zyklen amplifiziert wurde. Die Basenfolge der zweiten Sonde und des komplementären Liganden geht aus Tabelle 2 hervor. Die Messung des Probenansatzes ohne Hybridisierungsreaktion ergab eine Signaldifferenz von 601 RU, entsprechend einer Konzentration an zweiter Sonde von 4,30 mM. Die Messung des Probenansatzes mit Hybridisierung ergab eine Signaldifferenz von 366 RU, entsprechend einer Konzentration an zweiter Sonde von 2,49 mM. Damit sind 4,30 mM minus 2,49 mM = 1,81 mM der zweiten Sonde durch Hybridisierung verbraucht worden, entsprechend 1,81 mM Kontrollsequenz.

### Ermittlung des GVO-Anteils

Die 1,81 mM Kontrollsequenz werden gleich 100% gesetzt. Die 0,81 mM Zielsequenz entsprechen dann einem GVO-Anteil im Maismehl von 44,75%.

## Patentansprüche

1. Verfahren zur qualitativen und quantitativen Bestimmung von genetischem Material, das eine für das Material charakteristische, bekannte Zielsequenz enthält und neben oder im Gemisch mit anderem genetischen Material vorliegt, **dadurch gekennzeichnet, daß** man
eine Suspension, die das genetische Material mit der Zielsequenz sowie anderes genetisches Material enthält, mit (1) einem molaren Überschuß einer ersten Oligonukleotidsonde versetzt, deren Nukleotidsequenz zu der Zielsequenz komplementär ist, und (2) mit einem molaren Überschuß einer zweiten Oligonukleotidsonde versetzt, deren Nukleotidsequenz zu einer Nukleotidsequenz komplementär ist, die in allen Komponenten des gesamten genetischen Materials enthalten ist, auf die sich die Quantifizierung beziehen soll,
das gesamte genetische Material, soweit es doppelsträngig ist, einsträngig macht, die erste Oligonukleotidsonde mit der Zielsequenz und die zweite Oligonuleotidsonde mit der in allen Komponenten des genetischen Materials enthaltenen Nukleotidsequenz hybridisiert,
in der Suspension die Konzentrationen der nicht hybridisierten ersten und zweiten Oligonukleotidsonde bestimmt und aus den Konzentrationen den Anteil des genetischen Materials mit der Zielsequenz an dem gesamten genetischen Material, auf das sich die Quantifizierung beziehen soll, ermittelt.

2. Verfahren zur quantitativen Bestimmung der Konzentration von genetischem Material, das eine charakteristische, bekannte Nukleotidsequenz als Zielsequenz enthält und neben oder im Gemisch mit anderem genetischen Material vorliegen kann, **dadurch gekennzeichnet, daß** man
eine Suspension, die das genetische Material mit der Zielsequenz sowie gegebenenfalls anderes genetisches Material enthält, mit einem molaren Überschuß nur einer Oligonukleotidsonde versetzt, deren Nukleotidsequenz zu der Zielsequenz komplementär ist,
das genetische Material, soweit es doppelsträngig ist, einsträngig macht, die Sonde mit der Zielsequenz hybridisiert,
in der Suspension die Konzentration der nicht hybridisierten Sonde bestimmt und aus dieser Konzentration die Konzentration des genetischen Materials in der Suspension ermittelt.

3. Verfahren nach Anspruch 1 oder 2, **dadurch gekennzeichnet, daß** das genetische Material DNA, RNA, zum Teil DNA und zum Teil RNA, PNA oder eine andere modifizierte Basensequenz ist.

4. Verfahren nach einem der Ansprüche 1 bis 3, **dadurch gekennzeichnet, daß** die Oligonukleotidsonden aus DNA, RNA, PNA oder anderen modifizierten Basensequenzen bestehen.

5. Verfahren nach einem der Ansprüche 1 bis 4, **dadurch gekennzeichnet, daß** die Konzentrationen der beiden Sonden nach einem markierungsfreien Detektionsverfahren bestimmt werden.

6. Verfahren nach Anspruch 5, **dadurch gekennzeichnet, daß** das Detektionsverfahren auf Oberflächenplasmonenresonanz beruht.

7. Verfahren nach Anspruch 1 oder 3 bis 6, **dadurch gekennzeichnet, daß** die Hybridisierung der ersten und der zweiten Sonde in Einzelschritten mit Teilmengen desselben genetischen Materials durchgeführt wird.

8. Verfahren nach Anspruch 1 oder 3 bis 6, **dadurch gekennzeichnet, daß** die Hybridisierung der ersten und der zweiten Sonde mit dem genetischen Material in ein und demselben Reaktionsansatz durchgeführt wird und anschließend die überschüssigen, nicht gebundenen Sondenmoleküle bestimmt werden.

9. Verfahren nach einem der Ansprüche 1 bis 8, **dadurch gekennzeichnet, daß** die erste und gegebenenfalls die zweite Sonde nach der Hybridisierung durch immobilisierte Oligonukleotide aufgrund spezifischer Basenpaarung gebunden werden.

10. Verfahren nach Anspruch 9, **dadurch gekennzeichnet, daß** der immobilisierte Ligand ein Antikörper gegen ein Antigen ist, das auch an die Sondenmoleküle gebunden ist.

11. Verfahren nach Anspruch 9, **dadurch gekennzeichnet, daß** der immobilisierte Ligand ein Antigen ist, die Sonde mit dem gleichen Antigen verknüpft ist, die nicht hybridisierten Antigen-modifizierten Sondenmoleküle mit einem Überschuß an Antikörper versetzt werden und der überschüssige Antikörper durch eine Antigen/Antikörper-Reaktion mit dem immobilisierten Antigen auf dem Sensorchip gebunden wird.

12. Verfahren nach einem der Ansprüche 1 bis 11, **dadurch gekennzeichnet, daß** eine PCR vorgeschaltet wird.

13. Verfahren nach einem der Ansprüche 1 bis 11, **dadurch gekennzeichnet, daß** man die Molmasse der ersten und/oder zweiten Sonde vergrößert.

14. Verfahren nach Anspruch 13, **dadurch gekennzeichnet, daß** man die Molmasse von Antigen-modifizierten Sonden vergrößert, indem man einen gegen das Antigen gerichteten Antikörper auf sie einwirken läßt und gegebenenfalls die Molmasse des so vergrößerten Moleküls mit einem weiteren, gegen den ersten Antikörper gerichteten zweiten Antikörper weiter vergrößert.

15. Verfahren nach Anspruch 13, **dadurch gekennzeichnet, daß** man die Molmasse von gegebenenfalls Antigen-modifizierten Sonden vergrößert, indem man die Zahl der Nukleotide über etwa 20 hinaus auf bis zu etwa 70 vergrößert.

16. Verfahren nach einem der Ansprüche 13 bis 15, **dadurch gekennzeichnet, daß** man sowohl die erste als auch die zweite Sonde mit dem gleichen Antigen verknüpft.

17. Verfahren nach einem der Ansprüche 13 bis 16, **dadurch gekennzeichnet, daß** man einen Vorhybridisierungsschritt mit einem Bindungsvermittler durchführt, der eine zu der auf dem Sensorchip immobilisierten Nukelotidsequenz komplementäre Nukleotidsequenz sowie eine zu der Zielsequenz bzw. zu der Kontrollsequenz komplementäre Nukleotidsequenz aufweist, erste und zweite Sonden verwendet, die eine zu der noch freien Nukleotidsequenz des Bindungsvermittlers komplementäre Nukleotidsequenz haben, und die überschüssige erste und zweite Sonde mit dem Bindungsvermittler hybridisiert.

18. Verfahren nach einem der Ansprüche 5 bis 17, **dadurch gekennzeichnet, daß** man zusätzlich alternative erste und/oder zweite Sonden einsetzt, die mit anderen Basenfolgen der Zielsequenz bzw. der Kontrollsequenz hybridisieren.

## Claims

1. A method for qualitatively and quantitatively determining genetic material which contains a known target sequence which is characteristic for the material and which is present alongside, or in a mixture with, other genetic material, **characterized in that** a suspension which contains the genetic material possessing the target sequence and also other genetic material is treated with (1) a molar excess of a first oligonucleotide probe whose nucleotide sequence is complementary to the target sequence and (2) with a molar excess of a second oligonucleotide probe whose nucleotide sequence is complementary to a nucleotide sequence which is present in all the components of the total genetic material to which the quantification is to relate, the total genetic material, insofar as it is double-stranded, is rendered single-stranded,
the first oligonucleotide probe is hybridized with the target sequence and the second oligonucleotide probe is hybridized with the nucleotide sequence which is present in all the components of the genetic material,
the concentrations of the unhybridized first and second oligonucleotide probes are determined in the suspension and the proportion of the genetic material possessing the target sequence in the total genetic material to which the quantification is to relate is elucidated from the concentrations.

2. A method for quantitatively determining the concentration of genetic material which contains, as target sequence, a characteristic, known nucleotide sequence and can be present alongside, or in a mixture with, other genetic material, **characterized in that**
a suspension which contains the genetic material possessing the target sequence and also, where appropriate, other genetic material is treated with a molar excess of only one oligonucleotide probe whose nucleotide sequence is complementary to the target sequence,
the genetic material, insofar as it is double-stranded, is rendered single-stranded and the probe hybridizes with the target sequence,
the concentration of the unhybridized probe is determined in the suspension and the concentration of the genetic material in the suspension is elucidated from this concentration.

3. The method as claimed in claim 1 or 2, **characterized in that** the genetic material is DNA, RNA, partially DNA and partially RNA, PNA or another modified base sequence.

4. The method as claimed in one of claims 1 to 3, **characterized in that** the oligonucleotide probes consist of DNA, RNA, PNA or other modified base sequences.

5. The method as claimed in one of claims 1 to 4, **characterized in that** the concentrations of the two probes are determined using a label-free detection method.

6. The method as claimed in claim 5, **characterized in that** the detection method is based on surface plasmon resonance.

7. The method as claimed in claims 1 or 3 to 6, **characterized in that** the hybridization of the first probe and the second probe is carried out in individual steps using constituent quantities of the same genetic material.

8. The method as claimed in claims 1 or 3 to 6, **characterized in that** the hybridization of the first probe and the second probe with the genetic material is carried out in one and the same reaction mixture and the excess, unbound probe molecules are then determined.

9. The method as claimed in one of claims 1 to 8, **characterized in that** the first probe and, where appropriate, the second probe are bound, after the hybridization, by immobilized oligonucleotides as the result of specific base pairing.

10. The method as claimed in claim 9, **characterized in that** the immobilized ligand is an antibody which is directed against an antigen which is also bound to the probe molecules.

11. The method as claimed in claim 9, **characterized in that** the immobilized ligand is an antigen, the probe is linked to the same antigen, the unhybridized antigen-modified probe molecules are treated with an excess of antibody and the excess antibody is bound by means of an antigen/antibody reaction to the immobilized antigen on the sensor chip.

12. The method as claimed in one of claims 1 to 11, **characterized in that** it is preceded by a PCR.

13. The method as claimed in one of claims 1 to 11, **characterized in that** the molar mass of the first and/or second probe is increased.

14. The method as claimed in claim 13, **characterized in that** the molar mass of antigen-modified probes is increased by allowing an antibody which is directed against the antigen to act on them and, where appropriate, the molar mass of the molecule, which has been increased in this way, is further increased using another, second antibody which is directed against the first antibody.

15. The method as claimed in claim 13, **characterized in that** the molar mass of probes, which may, where appropriate, be antigen-modified, is increased by increasing the number of nucleotides beyond about 20 to up to about 70.

16. The method as claimed in one of claims 13 to 15, **characterized in that** both the first probe and the second probe are linked to the same antigen.

17. The method as claimed in one of claims 13 to 16, **characterized in that** a pre-hybridization step is carried out using a binding mediator which possesses a nucleotide sequence which is complementary to the nucleotide sequence which is immobilized on the sensor chip and a nucleotide sequence which is complementary to the target sequence and/or to the control sequence, use is made of first and second probes which have a nucleotide sequence which is complementary to the nucleotide sequence of the binding mediator which is still free and the excess first and second probes are hybridized with the binding mediator.

18. The method as claimed in one of claims 5 to 17, **characterized in that** use is additionally made of alternative first and/or second probes which hybridize with other base sequences of the target sequence or of the control sequence.

## Revendications

1. Procédé pour la détermination qualitative et quantitative de matériau génétique qui contient une séquence cible connue caractéristique pour le matériau et est disponible à côté de ou en mélange avec un ou plusieurs autres matériaux génétiques, **caractérisé en ce que**
on mélange une suspension qui contient le matériau génétique avec la séquence cible ainsi qu'un autre matériau génétique avec (1) un excédent molaire d'une première sonde oligonucléotidique dont la séquence nucléotidique est complémentaire à la séquence cible, et (2) avec un excédent molaire d'une deuxième sonde oligonucléotidique dont la séquence nucléotidique est complémentaire à une séquence nucléotidique qui est contenue dans toutes les composantes du matériau génétique entier et à laquelle la quantification doit se rapporter,
dans la mesure où le matériau génétique entier est un matériau à brin double, on en fait un matériau à un brin en hybridant la première sonde oligonucléotidique avec la séquence cible et la deuxième sonde oligonucléotidique avec la séquence nucléotidique contenue dans toutes les composantes du matériau génétique,
on détermine dans la suspension les concentrations des première et deuxième sonde oligonucléotidique non hybridées et détermine à partir des concentrations la part de matériau génétique avec la séquence cible par rapport au matériau génétique entier à laquelle doit se rapporter la quantification.

2. Procédé pour la détermination qualitative et quantitative de matériau génétique qui contient une séquence nucléotidique cible connue caractéristique comme séquence cible et peut être disponible à côté de ou en mélange avec un ou plusieurs autres matériaux génétiques, **caractérisé en ce que**
on mélange une suspension qui contient le matériau génétique avec la séquence cible ainsi que, le cas échéant, un autre matériau génétique, avec un excédent molaire d'une seule sonde oligonucléotidique dont la séquence nucléotidique est complémentaire à la séquence cible,
dans la mesure où le matériau génétique est un matériau à brin double, on en fait un matériau à un brin en hybridant la sonde avec la séquence cible,
on détermine dans la suspension la concentration de la sonde non hybridée et détermine à partir de cette concentration la concentration du matériau génétique dans la suspension.

3. Procédé selon la revendication 1 ou 2, **caractérisé en ce que** le matériau génétique est ADN, ARN, en partie ADN et en partie ARN, APN ou une autre séquence de base modifiée.

4. Procédé selon la revendication 1 à 3, **caractérisé en ce que** les sondes oligonucléotidiques se composent d'ADN, ARN, APN ou d'autres séquences de bases modifiées.

5. Procédé selon la revendication 1 à 4, **caractérisé en ce que** les concentrations des deux sondes sont déterminées selon un procédé de détection sans marquage.

6. Procédé selon la revendication 5, **caractérisé en ce que** le procédé de détection se base sur la résonance plasmonique de surface.

7. Procédé selon la revendication 1 ou 3 à 6, **caractérisé en ce que** l'hybridation de la première et la deuxième sonde est réalisée en étapes individuelles avec des quantités partielles du même matériau génétique.

8. Procédé selon la revendication 1 ou 3 à 6, **caractérisé en ce que** l'hybridation de la première et la deuxième sonde avec le matériau génétique est réalisée dans la seule et même formulation de réaction, puis les molécules de sondes excédentaires non liées sont déterminées.

9. Procédé selon l'une quelconque des revendications 1 à 8, **caractérisé en ce que** la première et, le cas échéant, la deuxième sonde sont liées après l'hybridation par des oligonucléotides immobilisés en raison d'appariement de bases spécifique.

10. Procédé selon la revendication 9, **caractérisé en ce que** le ligand immobilisé est un anticorps contre un antigène qui est également lié à la molécule de sonde.

11. Procédé selon la revendication 9, **caractérisé en ce que** le ligand immobilisé est un antigène, la sonde est reliée au même antigène, les molécules de sonde non hybridées et modifiées par antigène sont mélangées avec un excédent d'anticorps et l'anticorps excédentaire est lié par une réaction antigène/anticorps avec l'antigène immobilisé sur la puce de capteur.

12. Procédé selon l'une quelconque des revendications 1 à 11, **caractérisé en ce qu'**un système ACP est prévu en amont.

13. Procédé selon l'une quelconque des revendications 1 à 11, **caractérisé en ce qu'**on augmente la masse molaire de la première et/ou deuxième sonde.

14. Procédé selon la revendication 13, **caractérisé en ce que**, pour augmenter la masse molaire de sondes modifiées par antigène, on fait agir un anticorps orienté contre l'antigène sur ces dernières et, si nécessaire, augmente encore plus la masse molaire de la molécule ainsi agrandie avec un deuxième anticorps orienté contre le premier anticorps.

15. Procédé selon la revendication 13, **caractérisé en ce que**, pour augmenter la masse molaire de sondes éventuellement modifiées par antigène, on augmente le nombre de nucléotides à plus d'environ 20 jusqu'à environ 70.

16. Procédé selon l'une quelconque des revendications 13 à 15, **caractérisé en ce qu'**on relie aussi bien la première sonde que la deuxième sonde au même antigène.

17. Procédé selon l'une quelconque des revendications 13 à 16, **caractérisé en ce qu'**on réalise une étape de pré-hybridation avec un médiateur de liaison qui présente une séquence nucléotidique complémentaire à la séquence nucléotidique immobilisée sur la puce de capteur ainsi qu'une séquence nucléotidique complémentaire à la séquence cible ou la séquence de contrôle, utilise une première et une deuxième sonde qui ont une séquence nucléotidique complémentaire à la séquence nucléotidique encore libre du médiateur de liaison, et hybride les première et deuxième sondes hybridées avec le médiateur de liaison.

18. Procédé selon l'une quelconque des revendications 5 à 17, **caractérisé en ce qu'**on utilise en plus des première et/ou deuxième sondes alternatives qui s'hybrident avec d'autres séquences de bases de la séquence cible ou de la séquence de contrôle.
